# FASCICULE DE BREVET EUROPEEN

(11) **EP 1 082 083 B1**
(45) Date de publication et mention de la délivrance du brevet: **23.04.2003**
(21) Numéro de dépôt: 99920930.7
(22) Date de dépôt: 27.05.1999
(51) Int. Cl.: A61K 7/06

(54) **COMPOSITIONS CAPILLAIRES CONTENANT UN EXTRAIT DE PFAFFIA PANICULATA**
HAARPFLEGEMITTEL ENTHALTEND EINEN PFAFFIA PANICULATA EXTRAKTEN
HAIR COMPOSITIONS CONTAINING A PFAFFIA PANICULATA EXTRACT

(30) Priorité: 28.05.1998 FR 9806734
(43) Date de publication de la demande: 14.03.2001
(73) Titulaire: Pierre Fabre Dermo-Cosmetique, 92100 Boulogne-Billancourt (FR)
(72) Inventeur: FABRE, Bernard, F-31450 Belberaud (FR); CHARVERON, Marie, F-31000 Toulouse (FR); JEANJEAN, Michel, F-31320 Castanet-Tolosan (FR)
(74) Mandataire: Ahner, Francis
(86) Numéro de dépôt international: FR9901239
(87) Numéro de publication internationale: WO99060991

(56) Documents cités:
- WO-A-94/04132
- US-A- 5 198 225
- K. Schrader: "Grundlagen und Rezepturen der Kosmetika", 2me ed.; Hüthig Buch Verlag, Heidelberg (DE); p. 672 (1989)

## Description

La présente invention concerne des compositions cosmétiques ou dermatologiques destinées à prévenir et/ou traiter la chute des cheveux.

Les cheveux présentent un cycle de vie qui peut être différencié en trois grandes étapes : les phases anagène, catagène et télogène.

La première phase dite anagène est une période active de croissance qui dure trois à cinq ans. Durant cette phase, le cheveu grandit de 0,35 mm par jour. Vient ensuite la phase catagène ou phase de régression qui est en fait une période de transition. Le follicule pileux évolue, la kératogénèse s'interrompt. Cette phase est très rapide. Elle dure environ trois semaines. La dernière phase appelée phase télogène est une phase de repos et d'élimination qui dure en moyenne trois mois. Le cheveu reste ancré dans le follicule régressé puis tombe.

Après une phase de latence de deux à cinq mois, une nouvelle phase anagène se redéclenche.

La croissance de la tige pilaire durant la phase anagéne, dépend de la prolifération et des facteurs cytosolubles des cellules du bulbe folliculaire. L'expression de certains facteurs de croissance varie au cours du cycle pilaire. D'autre part, on note aussi un changement de la vascularisation en fonction du cycle pilaire. Si la croissance du poil dépend de plusieurs facteurs, les facteurs susceptibles d'induire une angiogénèse sont des candidats de choix dans l'induction et le maintien de la vascularisation au niveau de la papille dermique car on note une disparition du réseau capillaire de la papille durant le stade télogène.

Le facteur de croissance VEGF (Vaso Endothelial Growth Factor) est un facteur de croissance angiogénique mitogène sécrété par les cellules endothéliales vasculaires.

Les cellules de la papille dermique du follicule pileux présentent des récepteurs du facteur VEGF et le produisent in vitro (Lachgar et al., J. Invest. Dermatol., 1996, 107 : (17-23).

La protéine et les ARN messagers du VEGF, sont également exprimés au niveau des cellules de la papille dermique durant le stade anagène (Lachgar et al., In «Hair for the next millenium». Eds D.J.J. Van Neste, V.A. Randall, H. Baden, H. Ogawa, R. Oliver, Elsevier, Amsterdam, 1996, pp. 407-412).

L'activité angiogénique peut être due de façon spécifique à la production du VEGF. Elle peut également être observée après une stimulation du métabolisme des cellules endothéliales.

La Demanderesse a mis en évidence qu'un extrait végétal de Pfaffia paniculata présente une activité angiogénique.

Pfaffia paniculata (Martins) Kuntze est une plante appartenant à la famille botanique des Amaranthaceae. Cette plante est d'origine amazonienne et c'est au Brésil que l'on trouve une utilisation médicinale et en particulier des racines. En effet, ces dernières correspondent au Ginseng Brésilien qui est utilisé traditionnellement comme tonique, aphrodisiaque et comme antidiabétique. La forme des racines de Pfaffia paniculata se rapproche de celles de Panax Ginseng, ce qui lui vaut probablement son appellation vernaculaire de Ginseng Brésilien. L'autre nom vernaculaire utilisé est le Suma.

Les racines de Pfaffia paniculata sont décrites dans la littérature comme contenant des stérols: stigmastérol, sitostérol et leurs hétérosides, de l'allantoïne, des saponines nortriterpéniques avec l'acide pfaffique et ses hétérosides = pfaffosides A, B, C, D, E, F. Takemoto et al. (1983) : Tetrahedron letters 24, 10, 1057 - 1060, Nakai et al. (1984) : Phytochemistry, 23, 8, 1703 - 1705, Nishimoto et al. (1984) : Phytochemistry, 23, 1, 139- 142.

L'acide pfaffique et les pfaffosides correspondants sont décrits comme possédant des activités inhibitrices sur la croissance des mélanomes.

Ces saponines particulières s'apparentent aux ecdystéroïdes et à son chef de file, l'ecdysone.

Cette molécule et ses métabolites sont des hormones qui interviennent dans la régulation de la mue et d'autres phénomènes vitaux des insectes (reproduction, métamorphoses, ...).
Meybeck et al. (brevet US-5 609 873) ont décrit l'utilisation d'ecdystéroïdes dans des compositions dermatologiques et cosmétiques et en particulier pour maintenir l'effet barrière de la peau envers l'eau ou pour la préparation de milieu de culture de cellules cutanées. Ces molécules ont également été décrites comme pesticides (JP-72 74748) et comme analgésiques (JP-63 002928).

Enfin, Pfaffia paniculata et Pfaffia stenopylla sont décrits par Mazzanti et col. (1993) : Pharmacological Research 27, sup. 1, 91-92, Phytotherapy Research., (1994) : 8, 413 - 416) comme possédant des activités anti-inflammatoires. Cette activité serait à rapprocher, selon les auteurs, à la présence de saponines dans les extraits réalisés.

La présente invention concerne des compositions cosmétiques ou dermatologiques destinées à traiter et/ou à prévenir la chute des cheveux, contenant à titre d'ingrédient actif un extrait de Pfaffia paniculata, éventuellement associé à un excipient cosmétiquement ou dermatologiquement acceptable.

L'extrait de Pfaffia paniculata est de préférence un extrait hydroéthanolique.

Les compositions selon l'invention contiennent de 0,3 à 5 % en poids de l'extrait de Pfaffia paniculata.

L'extraction est réalisée sur les racines de Pfaffia préalablement broyées. Le broyage est effectué par des broyeurs à marteaux ou à couteaux. L'extraction est effectuée par des solvants polaires, un alcool de C1 à C4, de l'acétone, de l'eau ou un mélange d'acétone-eau ou alcool de C1 à C4 -eau dans des proportions variant de 100-0 à 0-100 en volume.

Le rapport plante/solvant varie de 1/4 à 1/20 en masse. L'extraction peut se faire sous agitation ou par lixiviation et les températures d'extraction varient de la température ambiante à l'ébullition du solvant d'extraction. La durée d'extraction se situe entre 1 heure et 24 heures.

Une fois l'extraction réalisée, les solutions sont récupérées par filtration, essorage ou centrifugation. Le marc peut être réextrait en reprenant les mêmes paramètres que précédemment. Les deux solutions sont alors jointes.

Les solutions recueillies peuvent être évaporées sous pression réduite aux températures d'ébullition des solvants aux pressions exercées. Cette concentration peut être menée complètement en combinaison avec une technique de séchage de façon à obtenir une poudre. Elle ne peut être que partielle jusqu'à l'obtention d'une solution aqueuse. Dans ce cas, un solvant est alors ajouté comme la glycérine, le propylène glycol, le butylène glycol ou le transcutol. Les teneurs en matières sèches de ces extraits varient alors de 15 à 1 %. Les proportions de solvant et d'eau se situent entre 10-90 à 90-10. Les extraits sont filtrés si nécessaires.

Les extraits sont analysés pour quantifier les saponines. Pour ce faire, le test à mousse décrit dans la Pharmacopée peut être mis en oeuvre.

L'acide pfaffique peut également être identifié par chromatographie sur couche mince après hydrolyse acide de l'extrait.

De plus, l'extrait est riche en sucre puisque sa teneur dosée par la méthode à l'anthrone peut s'élever suivant les solvants à 80 % par rapport à la matière sèche extraite. Le sucre majoritaire présent est le glucose.

La présente invention est illustrée de façon non limitative par les exemples suivants.

### EXEMPLE 1 : (exemple de référence)

100 kg de racines de Pfaffia sont broyés puis extraits par 700 litres de solution éthanolique à 60 % sous reflux et sous agitation durant 1 heure. La solution est récupérée après filtration puis concentrée sous pression réduite jusqu'à l'obtention d'une teneur en matière sèche de 10 %. La solution obtenue est diluée par du propylène glycol jusqu'à l'obtention d'une solution à 2 % de matière sèche. L'acide pfaffique est identifié par chromatographie sur couche mince et les sucres sont dosés entre 70 et 90 % par rapport à la matière sèche.

1 kg de racines est extrait après broyage par 10 kg d'acétone à froid et par lixiviation. La solution obtenue est évaporée jusqu'à l'obtention d'une poudre. L'acide pfaffique est identifié par chromatographie sur couche mince et la teneur en sucre évaluée entre 50 et 70 %.

10 kg de racines sont broyés puis extraits à 60°C avec 50 I d'un mélange eau-éthanol 60-40. La solution obtenue après filtration contient de l'acide pfaffique dans les 8 à 10 % de matière sèche obtenue.

Les sucres sont déterminés par réaction à l'anthrone entre 70 et 80 % de la matière sèche.

### EXEMPLE 2: activité sur le métabolisme cellulaire de cellules endothéliales (exemple de référence).

Le sel de tétrazolium de l'acide benzène sulfonique sodium 33 [1 (phenylamino) carbonyle] 3-4 tetrazolium] bis (4-methoxy-6-nitro) hydraté, communément appelé XTT, est couramment utilisé afin de détecter la viabilité cellulaire. Le XTT est réduit par les dihydrogénase mitochondriales des cellules endothéliales vivantes en présence d'un agent couplant d'électron, le coenzyme Q en un composé hydrosoluble jaune/orangé, le formozan. La quantité de formozan est fonction du nombre de cellules vivantes mais aussi, pour un nombre de cellules constant, de l'activité métabolique de la cellule.

Nous nous sommes intéressés à l'influence d'un extrait de Pfaffia sur la métabolisation du XTT par une population fixe de cellules endothéliales et ceci en concentration suboptimale de sérum donc des conditions d'activité métabolique faible.

A une concentration de 0,001 %, un extrait sec obtenu par extraction à l'alcool 60 % de Pfaffia stimule l'activité métabolique des cellules endothéliales de 48,4 % en 30 min, 75 % en 60 min et atteint un plateau à 75 min à 63 %. L'activité à 0,0002 % de concentration est légère puisqu'on observe une légère augmentation de 6 % de l'activité métabolique à 60 min.

### EXEMPLE 3: activité sur la production du VEGF (Vaso Endothelial Growth Factor) (exemple de référence)

L'activité angiogénique d'un extrait de Pfaffia a été évaluée sur la production de VEGF par les cellules de la papille dermique du follicule pileux.

Pour ce faire, une suspension de cellules est mise en contact avec une solution de milieu contenant différentes concentrations d'extrait de Pfaffia pendant 48 heures.

Ces cellules, après avoir été rincées, sont lysées. Les sumageants de lyse sont dosés en VEGF par Kit Elisa. Les résultats sont exprimés par rapport au taux de protéines totales par rapport à un témoin sans extrait.

L'extrait sec de Pfaffia obtenu par extraction à l'alcool à 60 % stimule la production de VEGF de :
- 36,3 % à 4 µg/ml,
- 65,6 % à 20 µg/ml, et
- 52 % à 40 µg/ml.

### EXEMPLE 4 : Shampooing transparent

| | |
|---|---|
| Extrait glycolique Pfaffia | 2 g |
| Les Na (sol. 28 %) | 35 g |
| Cocamido propyl bétaïne (sol. 30 %) | 10 g |
| Diméthyicone copolyol | 1 g |
| Hydrolysat de protéines de blé | 3 g |
| Diéthanolamide de coprah | 1 g |
| Polyquaternium 22 | 3,50 g |
| Parfum | QS |
| Conservateurs | QS |
| Acide citrique QS pH 6,5 | |
| Eau purifiée QSP | 100 g |

### EXEMPLE 5 : Shampooing nacré

| | |
|---|---|
| Extrait glycolique Pfaffia | 2 g |
| Panthénol | 0,50 g |
| Les Na (sol. 28 %) | 25 g |
| Cocoamphodiacétate (sol. 30 %) | 12 g |
| Polysorbate 20 | 4 g |
| PEG 7 glycéryl cocoate | 2 g |
| PEG 150 distéarate | 3 g |
| Base nacrante (sol. 20 %) | 7 g |
| Hydroxypropyltrimmonium guar | 0,50 g |
| Acide citrique QS pH 6,5 | |
| Parfum | QS |
| Conservateurs | QS |
| Eau purifiée | 100 g |

### EXEMPLE 6 : Crème après-shampooing

| | |
|---|---|
| Extrait glycolique Pfaffia | 5 g |
| Hydrolysat de protéines de soie (sol. 20 %) | 2 g |
| Stéapyrium chlorure | 1,50 g |
| Cyclométhicone | 1,50 g |
| Alcool cétostéarylique | 3 g |
| Hydroxypropyl cellulose | 0,30 g |
| Parfum | QS |
| Acide Citrique QS pH 4,5 | |
| Eau purifiée QSP | 100 g |

### EXEMPLE 7 : Lotion pour le cuir chevelu

| | |
|---|---|
| Extrait glycolique Pfaffia | 0,30 g |
| D-Panthénol | 0,050 g |
| Biotine | 0,025 g |
| Diméthicole copolyol | 0,50 g |
| Chlorure d'alkyltriméthylammonium | 0,05 g |
| Essence de mélaleuca | 0,05 g |
| Ethanol | QS |
| Eau purifiée QSP | 100 g |

### EXEMPLE 8 : Soin antichute

| | |
|---|---|
| Extrait glycolique Pfaffia | 1 g |
| Extrait de racine de germe de chêne | 2 g |
| Fractions terpéniques de pin | 0,50 g |
| Tripeptides d'origine végétale | 1 g |
| Vitamine B5 | 0,30 g |
| Vitamine B6 | 0,15 g |
| Biotine | 0,05 g |
| Gluconate de zinc | 0,05 g |
| Triglycérides éthoxyles | QS |
| PEG 200 QS | 10 g |
| Ethanol QS | 15 g |
| Eau purifiée QSP | 100 g |

### EXEMPLE 9 : Concentré antichute polyvégétal

| | |
|---|---|
| Extrait glycolique Pfaffia | 2,50 g |
| Extrait d'ortie dioique | 3 g |
| Extrait de quinquina | 1,50 g |
| Extrait de centella asiatica | 0,25 g |
| D-Panthénol | 0,25 g |
| Vitamine B8 | 0,05 g |
| Vitamine B6 | 0,20 g |
| Ethoxydiglycol | 5 g |
| Pyrrolydonecarboxylate de zinc | 0,10 g |
| Huiles essentielles orange/sauge | QS |
| Ethanol | 50 % vol. |
| Eau purifiée QSP | 100 g |

## Revendications

1. , Compositions cosmétiques ou dermatologiques destinées à traiter et/ou à prévenir la chute des cheveux, contenant à titre d'ingrédient actif un extrait de Pfaffia paniculata, éventuellement associé à un excipient cosmétiquement ou dermatologiquement acceptable.

2. Composition selon la revendication 1, **caractérisée en ce que** l'extrait de Pfaffia paniculata est un extrait hydroéthanolique.

3. Compositions selon la revendication 1 ou 2, **caractérisées en ce qu'**elles contiennent 0,3 à 5 % en poids de l'extrait de Pfaffia paniculata.

## Patentansprüche

1. Kosmetische oder dermatologische Zusammensetzungen, die dazu bestimmt sind, einen Haarausfall zu behandeln und/oder zu verhüten, und als aktiven Bestandteil einen Extrakt von Pfaffia paniculata, gegebenenfalls in Verbindung mit einem kosmetisch oder dermatologisch annehmbaren Hilfsstoff, enthalten.

2. Zusammensetzung nach Anspruch 1, **dadurch gekennzeichnet, dass** der Extrakt von Pfaffia paniculata ein wässrig-ethanolischer Extrakt ist.

3. Zusammensetzungen nach Anspruch 1 oder 2, **dadurch gekennzeichnet, dass** sie 0,3 bis 5 Gew.-% Extrakt von Pfaffia paniculata enthalten.

## Claims

1. Cosmetic or dermatological compositions for treating and/or preventing hair loss, containing as active ingredient an extract of Pfaffia paniculata, optionally combined with a cosmetically or dermatologically acceptable excipient.

2. Composition according to Claim 1, **characterized in that** the extract of Pfaffia paniculata is an aqueous-ethanolic extract.

3. Compositions according to Claim 1 or 2, **characterized in that** they contain from 0.3% to 5% by weight of extract of Pfaffia paniculata.
